# EUROPEAN PATENT APPLICATION

(11) **EP 2 146 208 A1**
(43) Date of publication of application: **20.01.2010**
(21) Application number: 07713854.3
(22) Date of filing: 06.02.2007
(51) Int. Cl.: G01N 33/574

(54) **KIT FOR DECIDING DEGREE OF MALIGNANCY IN PROSTATE CANCER AND METHOD OF USING THE SAME**

(71) Applicant: J-Pharma Co., Ltd., Tokyo 160-0022 (JP)
(72) Inventor: ENDOU, Hitoshi, Sagamihara-shi Kanagawa 229-0022 (JP); OKAYASU, Isao, Sagamihara-shi Kanagawa 228-0803 (JP); SAKATA, Takeshi, Komae-shi Tokyo 201-0002 (JP)
(74) Representative: Giovannini, Francesca
(86) International application number: PCT/JP2007/052025
(87) International publication number: WO 2008/096416

(57) **Abstract**

The present invention relates to kits and methods for determining (diagnosing) prostate cancer malignancy and to predict patient prognoses. In addition to the Gleason's classification and the TMN classification, the invention kit and methods provide improved procedures with molecular markers. These new diagnostic methods provide methods capable of determining prostate cancer malignancy more accurately and easily through combination with the Gleason's classification via biopsies at an early stage before surgical operation; even when specimens taken through a fine needle examination are used instead of specimens extracted during a surgical operation.

## Description

### TECHNICAL FIELD

The present invention relates to kits and methods for determining (diagnosing) prostate cancer malignancy and predicting prognoses in patients.

### BACKGROUND ART

An oncofetal gene, the LAT1 gene was isolated by Kanai et al. in 1988 (Kanai, Y., Segawa, H., Miyamoto, K., Uchino, H., Takeda, E., Endou, H.: Expression Cloning and Characterization of a Transporter for Large Neutral Amino Acids Activated by the Heavy Chain of 4F2 Antigen. J. Biol. Chem. 273(37): 23629-23632, 1998). LAT1 genes code 44kD, 12 trans-membrane-spanning proteins having functions of L-type amino acid transporters; they exhibit activities of the classical transporter system L which transports large, neutral amino acids such as leucine, isoleucine, valine, phenylalanine, tyrosine, tryptophan, methionine and histidine in an Na⁺-independent manner under the coexistence of trans-membrane protein 4F2hc (also referred to as CD98). LAT1 and 4F2hc proteins are believed to be linked through a cysteine-cysteine linkage. Although LAT1 genes are highly expressed in cancer-derived culture cells and fetal livers, in normal tissues, they are only expressed in limited regions such as brain, placenta, testicle and bone marrow (Yanagida, O., Kanai, Y., Chairoungdua, A., Kim, D, Kyung., Segawa, H., Nii, T., Cha, S, Ho., Matsuo, H., Fukushima, J., Fukusawa, Y., Tani, Y., Taketani, Y., Uchino, H., Kim, J, Young., Inatomi, J., Okayasu, I., Miyamoto, K., Takeda, E., Goya, T., Endou, H.: Human L-type amino acid transporter 1 (LAT1): characterization of function and expression in tumor cell lines. Biochimica et Biophysica Acta 1514: 291-302, 2001). On the contrary, 4F2hc genes are extensively found in almost all normal tissues, cancer-derived cells and the like.

LAT2, a homolog of LAT1 (Segawa, H., Fukasawa, Y., Miyamoto, K., Takeda, E., Endou, H., Kanai, Y.: Identification and Functional Characterization of a Na+-independent Neutral Amino Acid Transporter with Broad Substrate Selectivity. J. Biol. Chem 274(28): 19745-19751, 1999) has 50% amino acid homology with LAT1 protein. Human tissue distribution of LAT2 genes was found in all tissues investigated. Thus, LAT1 may be regarded as an oncofetal L-type amino acid transporter and LAT2 as a normal L-type amino acid transporter. 4F2hc, an activator for LAT, has been identified in most tissues regardless if they are cancerous or normal.

Incidentally, prostate cancer has high occurrence throughout the world; in the United States, prostate cancer ranks first in morbidity and second in mortality among male cancers. Even in Japan, the number of patients with prostate cancer has increased in recent years; approximately 2,000 a year in 1975 increased to approximately 20,000 in 2000. It has been predicted that this number will increase to approximately 80,000 in 2020, ranking second in morbidity only to lung cancer among male cancers. Furthermore, mortality prediction due to prostate cancer will increase 1.4-fold by 2020 and that, in the United States, the mortality rate will rank first (Nippon Rinsho, extra edition 60; 44-48, 200; Clinical of Prostatic Diseases).

A well known method, PSA blood levels have been used as a method for early detection and screening of prostate cancer. However, PSA has been shown to not be specific to cancers and known to increase in prostatic hypertrophy and/or prostatitis. In recent years, it has been reported that PSA blood levels are proportional to prostate gland size. Commonly, after a high blood PSA level has been detected, the size, stiffness, and the like of the prostate gland are examined via rectal examination. Subsequently, if the clinician suspects prostate cancer, biopsies are taken and histopathologically diagnosed by means of hematoxylin eosin staining; cells are examined on the basis of atypism, differentiation and the like to determine their malignancy. Among histopathological diagnoses, the Gleason's classification has an atypism-based classification specific to prostate cancer; cancers are classified into five stages with scores of 1 to 5 according to the degree of atypism. It has, in recent years, become a diagnostic method which greatly influences the determination of therapies.

As a diagnostic method utilizing specimens extracted during a surgical operation, a diagnosis based on TMN (T: primary tumor, N: lymph node metastasis, M: remote metastasis) classification are supposed to be effective.

These diagnostic methods, especially the Gleason's classification, are widely used as methods for prognostication of prostate cancer and are reportedly relevant to prognoses. In the related field, however, in addition to Gleason's and TMN classification, more accurate methods to diagnose and treat prostate cancer, especially molecular markers are required. In addition, diagnostic methods such as the TMN classification which utilize specimens extracted during surgical operations are not very efficient in early diagnosis of malignancy of cancers.
Patent Reference 1: Japanese Unexamined Patent Publication No. 1999-299489
Patent Reference 2: Japanese Unexamined Patent Publication No. 2000-157286

### DISCLOSURE OF THE INVENTION

### PROBLEMS TO BE SOLVED BY THE INVENTION

An object of the present invention, in addition to Gleason's and TMN classification, provide procedures using molecular markers as new diagnostic methods and also to provide means capable of determining prostate cancer malignancy more accurately and easily through combination with the Gleason's classification using biopsies at an early stage before a surgical operation, even when specimens taken through a fine needle examination are used instead of specimens extracted during a surgical operation.

### MEANS FOR SOLVING THE PROBLEMS

In order to solve the above problem, the inventors first focused attention on amino acid transporter LAT1 which is expressed specifically to cancer-derived culture cells and fetal livers. Then, in studying various procedures for using LAT1 as a molecular marker to elicit LAT1, the inventors found that a procedure is specifically effective in determining malignancy of prostate cancer cells, with the use of samples taken by means of fine needle examination, to successfully accomplish the present invention.

The present invention (1) is a kit for determining malignancy of prostate cancer by means of immunohistochemical staining, which comprises an anti-human LAT1 monoclonal antibody. Herein, such anti-human LAT1 monoclonal antibodies are not particularly limited as long as they can specifically recognize LAT1; examples of which may include antibodies which specifically recognize amino acid residues at positions 1 to 52 from the N-terminus of the intracellular region of LAT1 (Met Ala Gly Ala Gly Pro Lys Arg Arg Ala Leu Ala Ala Pro Ala Ala Glu Glu Lys Glu Glu Ala Arg Glu Lys Met Leu Ala Ala Lys Ser Ala Asp Gly Ser Ala Pro Ala Gly Glu Gly Glu Gly Val Thr Leu Gln Arg Asn Ile Thr Lue) (for example, human LAT1 mouse monoclonal antibodies). The amino acid sequence and the base sequence of human LAT1 are described in Japanese Unexamined Patent Publication No. 2000-157286. In addition, in the context of the term "malignancy" as used herein, cancer has severe malignancy when a patient dies due to prostate cancer and mildly malignant when a patient, even if diagnosed with cancer, does not die directly due to prostate cancer.

Herein, anti-human LAT1 monoclonal antibodies are not particularly limited as along as they take LAT1 as antigens and bind to such antigens. Therefore, mouse antibodies, rat antibodies, rabbit antibodies, sheep antibodies and the like may appropriately be used.

Also, hybridomas producing monoclonal antibodies can be produced, basically using known techniques as follows. Specifically, monoclonal antibodies may be produced by using desired antigens and/or cells expressing such desired antigens as sensitized antigens, immunizing them according to conventional immunization methods, fusing the obtained immunocytes with known parent cells by means of conventional cell fusion methods and screening monoclonal antibody-producing cells (hybridomas) by means of conventional screening methods. Production of hybridomas may be carried out, for example, according to the method of Milstein et al. (Kohler, G. and Milstein, C., Methods Enzymol. (1981) 73: 3-46), and the like. In producing anti-human LAT1 monoclonal antibodies, LAT1 or fragments of the protein may be used as antigens; thus, LAT1 or cells expressing fragments of the protein may also be used as antigens. LAT1 or fragments of the protein may be obtained, for example, according to the method described in Molecular Cloning: A Laboratory Manual, 2nd. Ed., Vols. 1-3, Sambrook, J. et al, Cold Spring Harbor Laboratory Press, New York, 1989. Also LAT1 or cells expressing fragments of the protein may be obtained according to the method described in Molecular Cloning: A Laboratory Manual, 2nd. Ed., Vols. 1-3, Sambrook, J. et al, Cold Spring Harbor Laboratory Press, New York, 1989.

The kit may also include additional components, such as:

(1) an antibody labeled with peroxidase for anti-human LAT1 monoclonal antibody,
(2) a peroxide which inhibits endogenous peroxidase,
(3) a redox dye which develops a color via oxidization,
(4) an activator reagent for facilitating bonding between an antigen protein (LAT1) and an antibody,
(5) a blocking reagent which inhibits nonspecific bonding between proteins other than LAT1 in tissues and an antibody, and
(6) a cleaning agent for removing reagents attached to specimens at each step.

Regarding redox dye (3; above), while there are a number of signals whose intensities may be measured (for example, fluorescence), color changes in the visible light region are required. Reasons for this are not clear, but in case of other signals, use of the anti-human LAT1 monoclonal antibody according to the present invention does not provide sufficient distinction between benign versus malignant prostate cancer. On the other hand, using the anti-human LAT1 monoclonal antibody according to the present invention in combination with a reagent which enables observation of color changes within visible light regions (i.e. immunohistochemical staining), distinction between benign and malignant prostate cancer may clearly be defined.

The present invention (2) is a method to determine prostate cancer malignancy by means of immunohistochemical staining, which comprises a step of applying an anti-human LAT1 monoclonal antibody to a specimen tissue.

Herein, the method may additionally include any or all of the following steps:
a step of applying a peroxide to the specimen tissue,
a step of immersing the specimen tissue in an activator reagent and applying microwave treatment,
a step of applying a blocking reagent to the specimen tissue,
a step of applying a labeled antibody for the anti-human LAT1 monoclonal antibody,
a step of applying a redox dye which develops a color via oxidization, and
optionally, a step of applying a primary antibody negative control to the specimen tissue.

The present invention (3) also is a method of differentiating prostate cancer cases via application of LAT1 molecular target therapeutic agent(s), which comprises a step of determining prostate cancer malignancy according to the method of the invention (2) and a step of determining whether or not a therapeutic agent for prostate cancer be administered based upon the diagnosis result.

### BEST MODE FOR CARRING OUT THE INVENTION

The diagnostic agent according to the best mode employs a reagent kit for use in in-vitro diagnosis for differentiating prostate cancer cells and diagnosing the degree of proliferation (malignancy) of the cancer cells. The kit is composed of six reagents including a primary antibody, as a principal reactive agent. To sum up the determination principle, LAT1 expressed in cancer cells have 12 trans-membrane-spanning proteins with 507 amino acid residues. LAT1 can transport essential amino acids required for cell proliferation. The primary antibody (anti-human mouse monoclonal IgG antibody) used in the diagnostic method according to the best mode has an antibody which specifically recognizes amino acid residues at positions 1 to 52 from the N-terminus of the intracellular region of LAT1. As a result of searching the NCBI database, no other known proteins other than LAT1 are found to have the same sequence in human genes.

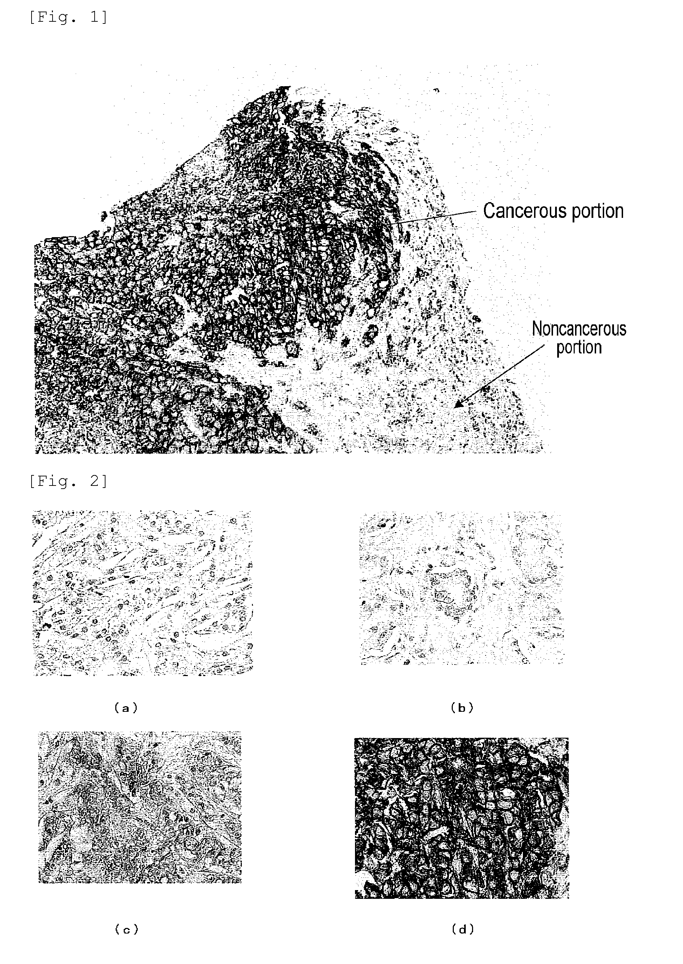

Specimens to be examined via the diagnostic agent according to the present invention are tissue sections of formalin-fixed, paraffin-embedded specimens from prostate cancer biopsies. Detection methods are conducted according fundamental immunohistochemical procedures using formalin-fixed, paraffin-embedded pathological tissue specimens such as those provided for ordinary pathological diagnoses. Specifically, after a paraffin-embedded specimen reacts with the antibody, a polymer reagent (secondary antibody) and staining reagent(s) are used for staining and subsequently observed through an optical microscope (Fig. 1). In Fig. 1, the stained portions are malignant cancer cells. To describe the principle of detection, LAT1 expressed in the membranes are bonded with the anti-LAT1 antibody (primary antibody), which further forms a complex with the polymer reagent. Furthermore, HRP of the polymer reagent reacts with the coloring reagent to develop a brown color. Since the antibody has very high affinity with LAT1, the present method provides very sensitive detection and selectivity. The method allows one to differentiate cancer cells from initial cancers (with initial expression of LAT1) versus advanced cancers (with overexpression of LAT1); furthermore, the method allows one to semi-quantitatively identify LAT1 expression and evaluate tumor (malignancy) progression.

### EXAMPLES

### Production Example 1 Example of Primary Antibody Production

The primary antibody (2.0 µg protein/ml) contains anti-human L-type amino acid transporter 1 (hLAT1) mouse monoclonal antibody. The antibody was made by using the proteins at positions 1 to 52 of hLAT1 synthesized by hLAT1 cloning vectors according to the in vitro translation method as antigens to immunize BALB/c mice and then fusing their spleen cells with mouse myeloma cells to obtain hybridomas, which were intraperitoneally inoculated to mice to obtain ascites fluid, which was purified by ammonium sulfate fractionation and Protein G coupling column chromatography and dissolved in 10 mM PBS (pH 7.4) containing 1% bovine serum albumin. The LAT1 amino acid sequence and the base sequence coding the protein are described in Japanese Unexamined Patent Publication No. 2000-157286.

### Production Example 2 Example of Determination Kit Composition

The determination kit according to the present invention is composed of the following six reagents.
Blocking reagent; prepared by diluting normal swine serum to 2%.
Primary antibody; prepared by diluting an anti-LAT1 mouse monoclonal antibody (Production Example 1) to 2 µg/mL with a buffer (1% BSA, 0.25% casein sodium, 15 mM sodium azide, 0.1% Tween 20).
Polymer reagent; Nichirei Histofine Simple Stain MAX-PO(M)(TM). This reagent contains 4 µg/mL of peroxidase-labeled anti-mouse IgG goat polyclonal antibody (Fab').
Primary antibody negative control; Mouse IgG (Vector Laboratories) gets dissolved in the buffer described above to 2 µg/mL.
Substrate buffer; Tris[hydroxyl methyl]amino methane and tris[hydroxyl methyl]amino methane are diluted with distilled water; and,
Coloring substrate; DAB (3-3'Diaminobendine tetrahydrochloride) dissolved in a buffer (substrate buffer described above) to 0.2 mg/mL.

The determination kit according this Production Example may further contain the following reagents used for staining.

### Endogenous peroxidase blocking reagent: 1% H₂O₂/methanol

Aqueous hydrogen peroxide is diluted with methanol to 1%.

### Activator reagent: 0.01 M citrate buffer (pH 6.0)

Citric acid monohydrate (0.36 g) and trisodium citrate dihydrate (2.44 g) are dissolved in distilled water to 1 L.

### Cleaning solution: PBS

Disodium hydrogen phosphate 12-water (2.90 g), sodium dihydrogen phosphate dihydrate (0.296 g) and sodium chloride (8.5 g) are dissolved in distilled water to 1 L.

To sum up the above, the reagents composing the diagnosis kit according to this Production Example (six essential reagents) are shown in Table 1 below.

**Table 1 Example of reagents composing the diagnosis kit according to this Production Example**

| Reagents | Kit for manual method | Kit for automatic immunostaining device |
|---|---|---|
| Blocking reagent (2% swine serum) | 7.0 mL | 2×11 mL |
| Primary antibody (containing 2 µg/mL of anti-hLAT1 antibody) | 3.5 mL | 1×15 mL |
| Polymer reagent (Simple Stain MAX PO(M)) | 7.0 mL | 2×11 mL |
| Primary antibody negative control (mouse IgG) | 3.5 mL | 1×15 mL |
| Substrate buffer (Tris buffer) | 10 mL | 15×11 mL |
| coloring substrate (0.2 mg/mL DAB solution) | 0.5 mL | 2×2 mL |

### Method for Operation and Method for Determination

### 1. Method for Operation

Procedures for operation are summarized in Table 2.

**Table 2 Immunohistochemical detection system**

| Steps | | Reagents | Operations |
|---|---|---|---|
| 1 | Endogenous peroxidase activation | 1% H₂O₂ | Treat for 30 min at room temperature under moist conditions |
| 2 | Antigen activation | Antigen activator solution | Microwave for 5 min and then treat for 20 min at room temperature |
| 3 | Blocking | Blocking reagent | Treat for 30 min at room temperature under moist conditions |
| 4 | Primary antibody treatment | Refer to Matters to be Considered | Treat for 1 hr at room temperature under moist conditions |
| 5 | Secondary antibody treatment | Nichirei Histofine Simple Stain MAX PO(M) | Treat for 30 min at room temperature under moist conditions |
| 6 | DAB coloring | Coloring substrate solution | Treat for 15 min at room temperature |
| 7 | counter-staining | Hematoxylin | Treat for 1 min at room temperature and then wash with water |

### 1-1. Method for Manual Operation

After deparaffinization, a specimen tissue slide is immersed in an endogenous peroxidase blocking reagent in a staining vat, treated for 30 minutes at room temperature and then washed with water. Excess moisture is removed from the specimen and the specimen is immersed in an activator reagent and then microwaved for five minutes. After the treatment, the specimen is sufficiently cooled down to room temperature and then washed with water and further with a cleaning solution. Excess moisture is removed from the specimen and a sufficient amount of blocking reagent to be uniformly distributed is added dropwise to the tissue section and allowed to react for 30 minutes at room temperature in a moist chamber. Excess moisture is removed from the specimen and a sufficient amount of primary antibody is added dropwise and allowed to react for one hour at room temperature in a moist chamber, followed by washing with a cleaning solution (three times each for five minutes). To a specimen tissue slide for negative control, a sufficient amount of primary antibody negative control is added dropwise, instead of the primary antibody, for similar treatment. Excess moisture is removed from the specimen and a sufficient amount of polymer reagent is added dropwise and allowed to react for 30 minutes at room temperature in a moist chamber, followed by washing with a cleaning solution (three times each for five minutes). Excess moisture is removed from the specimen and a predetermined amount of substrate solution is added dropwise to or immersed in the specimen and allowed to react for 15 minutes at room temperature in a moist chamber or staining pot, followed by washing with a cleaning solution. The specimen is stained with a counterstaining liquor (for example, Mayer's hematoxylin liquor) followed by washing with water. After dehydration with an alcohol series and substitution with xylene, the specimen is mounted for use in microscopy.

### 1-2. Method for Operation with Automatic Immunostaining Device

A specimen tissue slide, blocking reagent, primary antibody, primary antibody negative control, polymer reagent, substrate solution, distilled water, cleaning solution and counterstaining liquor are placed at predetermined locations and the reagents are allowed to react for a predetermined period of time at room temperature under moist conditions. Water of the specimen is substituted with an alcohol and then with xylene and the specimen is then mounted for use in microscopy.

### 2. Method for Determination

Overall staining is observed at a low magnification (×4, objective) and then the most intensely stained portions among tumor tissues in the specimen tissues are observed at higher magnifications (×10 to 40, objective) for scoring according to the following criteria (Fig. 2).

### a) Score 0

### LAT1 overexpression: not observed

Staining pattern: no cells are found as LAT1-positive in the membranes in the tumor tissues (Fig. 2a).

### b) Score 1

### LAT1 overexpression: not observed

Staining pattern: multiple cells are found which are LAT1-positive, but with low staining intensity, in the membranes in the tumor tissues (Fig. 2b).

### c) Score 2

### LAT1 overexpression: not observed

Staining pattern: multiple cells are found as moderately LAT1-positive, with staining intensity between Scores 1 and 3, in the membranes in the tumor tissues (Fig. 2c).

### d) Score 3

### LAT1 overexpression: observed

Staining pattern: multiple cells are found as highly LAT1-positive in the membranes in the tumor tissues, the whole membrane of each tumor cell having vivid wireloop-like staining (Fig. 2d).

### Test Example 1 Quality Confirmation Testing

In order to confirm the quality of the determination kit, a) specificity testing, b) sensitivity testing and c) simultaneous reproducibility testing were carried out on a control slide (prostate cancer-derived culture cells exhibiting LAT1 protein overexpression, PC3 cell lines) according to the following method. The subsequent Test Examples, including this Test Example, were carried out basically under the conditions described in Production Example 2 and Method for Determination, unless otherwise noted.

### a) Specificity Testing

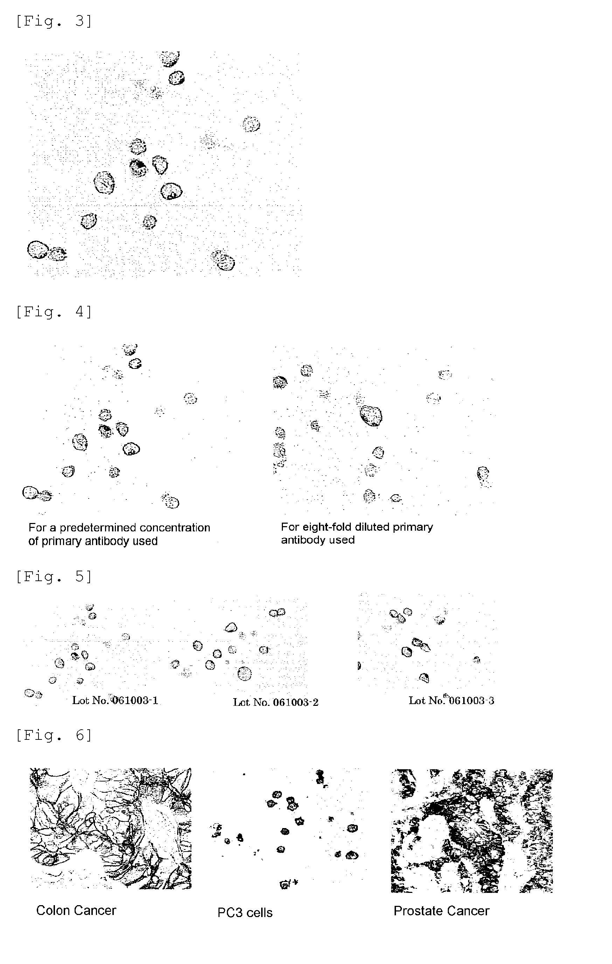

Using the determination kit of Production Example 2, a control slide (paraffin section specimen of prostate cancer-derived culture cells PC3) was stained according to Method for Operation described above to compare and study the results of staining of the primary antibody and the primary antibody negative control in the determination kit. The results of staining were rated as Score 0 to Score 3 on the basis of the criteria of Method for Determination described above (Table 3). For any of the test specimen kits, the control slide resulted in Score 3 (Fig. 3).

**Table 3 Results of staining using primary antibody**

| Specimen | Membrane staining | Cytoplasm staining | Nucleus staining | Background staining |
|---|---|---|---|---|
| Control slide (PC3-000000) | +++ | + | - | - |

Based on the results above, it was determined that using a primary antibody, specifically stained images were observed corresponding to LAT1 protein expression of each pathological tissue specimen to be tested, with no problems in staining behavior of the test specimens.

### b) Sensitivity Testing

Using the determination kit of Production Example 2, with the primary antibody in the determination kit as a control, the primary antibody solution in the test specimen kit that was diluted eight-fold with an antibody diluent was used to stain a management specimen slide and control slide according to Method for Operation described above and the primary antibody in the test specimen kit and the primary antibody that was diluted eight-fold were used to stain to compare and study the results. The results of staining were rated as Score 0 to Score 3 on the basis of the criteria of Method for Determination described above (Table 4 and Table 5). When a solution of primary antibody at a predetermined concentration was used, staining intensity of Score 3 was observed for the membranes of the control slide and, when a predetermined concentration of primary antibody that was diluted eight-fold with an antibody diluent was used, staining intensity of the membranes of the control slide decreased to Score 1 (Fig. 4).

**Table 4 Results of sensitivity testing**

| For typical primary antibody used | | | | |
|---|---|---|---|---|
| Specimen | Membrane staining | Cytoplasm staining | Nucleus staining | Background staining |
| Control slide (LS-180-000000) | +++ | + | - | - |

**Table 5**

| For eight-fold diluted primary antibody solution used | | | | |
|---|---|---|---|---|
| Specimen | Membrane staining | Cytoplasm staining | Nucleus staining | Background staining |
| Control slide (LS-180-000000) | + | + | - | - |

Based on the results above, since it was observed that when a predetermined concentration of primary antibody in a kit component was used, both control slides had Score 3 and when the predetermined concentration of primary antibody was diluted eight-fold, the LAT1 scores decreased. It was confirmed that the kit had a range of sensitivity within which the presence or absence of LAT1 protein expression in pathological tissue specimens can be determined.

### c) Simultaneous Reproducibility Testing

Using the determination kit of Production Example 2, three specimen tissue slides for management and three control slides were stained according to Method for Operation described above to compare and study the results of staining. The results of staining were rated as Score 0 to Score 3 on the basis of the criteria of Method for Determination described above (Table 6). All three control slides showed similar staining behaviors and were given similar scores (Fig. 5).

**Table 6 Results of staining three slides made from identical block using identical kit**

| (Experiment 1) | | | | | |
|---|---|---|---|---|---|
| Experiments | Specimens | Membrane staining | Cytoplasm staining | Nucleus staining | Background staining |
| 1 | Control slide (PC3-000000) | +++ | + | - | - |
| 2 | Control slide (PC3-000000) | +++ | + | - | - |
| 3 | Control slide (PC3-000000) | +++ | + | - | - |

Based on the results above, since when the three identical control slides were simultaneously stained, clear specific staining was observed in all the kits of three lots. Because their scores were similar, it was determined that there were no problems in simultaneous reproducibility and lot-to-lot difference.

### Test Example 2 Confirmation Testing of Staining Capability Using Cancer Tissue Specimens and Culture Cell Specimens

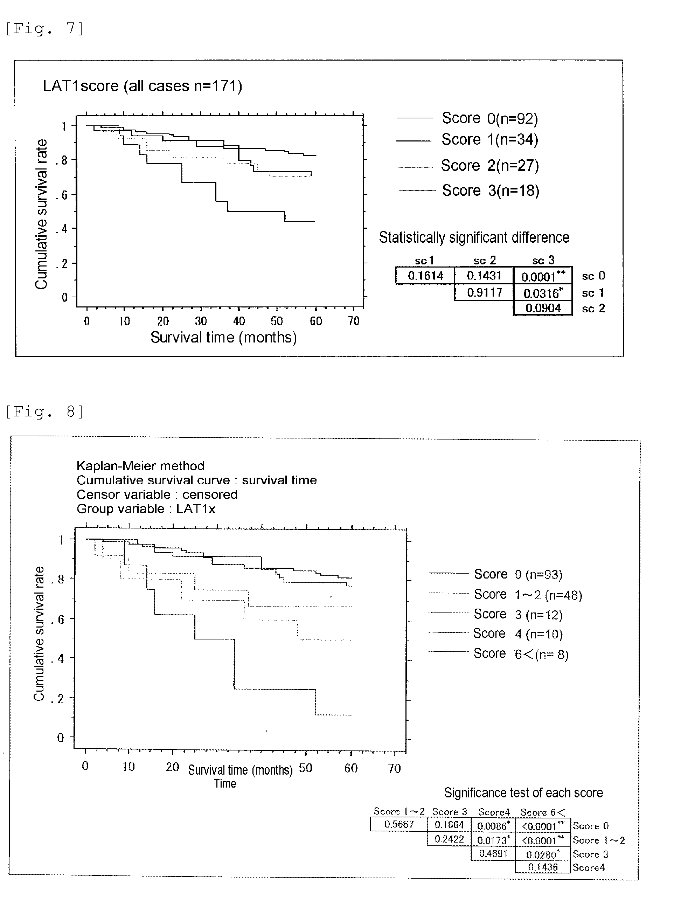

Using the determination kit of Production Example 2, confirmation testing for staining capability was carried out on prostate cancer-derived culture cells PC3 and a biopsy specimen of prostate cancer (both formalin-fixed, paraffin-embedded section specimens) as the target organ for this occasion. For reference, confirmation testing was also carried out on a colon cancer specimen extracted during a surgical operation, in which LAT1 is known to be highly expressed. This Test Example was carried out basically under the conditions described in Production Example 2 and Method for Determination described above, except the concentration of primary antibody shown in Fig. 7 below.

**Table 7 Matters to be considered**

| Matters to be considered | |
|---|---|
| Primary antibody | Primary antibody material of Production Example 1 Prepared by diluting with an antibody diluting buffer (see below) to 5 µg/mL. |

**Table 8 Specifics on pathological tissues used**

| | Pathological tissues | LAT1 protein expression rate* |
|---|---|---|
| Pathological tissues used | Colon cancer patient tissue No. E0502525 | +++ |
| | Culture cell PC3 No. 050302 | +++ |
| | Prostate cancer patient tissue No. 9401994 | +++ |

| | | |
|---|---|---|
| *Scores rated according to an immunohistochemical staining method using a stock solution of primary antibody | | |

The results are shown in Table 9 and Fig. 6.

**Table 9 Confirmation of staining capability by anti-LAT1 antibody using various specimens**

| Specimens used | Tumor cells | | | Background staining | Comment |
|---|---|---|---|---|---|
| | Membrane staining | Cytoplasm staining | Nucleus staining | | |
| Colon cancer | +++ | - | - | None | All showed intense |
| PC3 cells | +++ | - | - | - | staining |
| Prostate cancer | +++ | + | - | Interstitial portions stained | with Score 3 in membranes |

Based on the results above, wireloop-like intense staining was observed in the cancer cell membranes in all the specimens, confirming that high expression of LAT1 in cancer cells can be observed by means of immunohistochemical staining using the antibody.

### Test Example 3 Clinical Trial

We thought that demonstration from the viewpoint of clinical medicine was necessary and attempted to develop this testing. For cases in which surgical operations for prostate cancer were carried out, immunohistochemical staining of LAT1 was carried out using biopsies taken before the surgical operations.

After biopsying, slices prepared from the biopsies, stored as formalin-fixed, paraffin-embedded blocks, were stained using the determination kit of Production Example 2 and finally rated according to the intensity of brown, benzidine-stained images (Score 0 to 3). All these cases were divided into four groups with Scores 0 to 3 and statistical analysis was carried out according to the Kaplan-Meier method in which survival rate is plotted along the ordinate and passage of time (in month) is plotted along the abscissa. As a result, a significant difference was observed in malignancy of cancer between the case group with Score 0 and the case group with Score 3 on the basis of five-year survival rate. Also, although no significant difference was observed for Scores 1 and 2, survival rates dependent on respective Scores were shown. Thus, it was made possible to use this method as a new technique in diagnosing malignancy of cancers.

Furthermore, if antibody pharmaceuticals specific to LAT1 protein are developed in the future and if depressants (low molecular weight compounds) specific to LAT1/4F2hc functions are discovered, quantification of the presence of LAT1 to be partners (target molecules) for such pharmaceuticals and depressants suggests usefulness in discrimination of applicable cases for therapeutic agents.

### 1) Trial Sites and Number of Cases

Trial sites are two institutions A and B, each having the number of cases shown in the table below. Cases were independently extracted from each institution and subjected to testing.

**Table 10 Trial sites and number of cases**

| Trial sites | Physicians in charge | Subject patients and number of subject patients | Test periods |
|---|---|---|---|
| Institution A | | 164 cases | Mar. 1, 2006 to Dec. 12, 2006 |
| Institution B | | 7 cases | Nov. 1, 2006 to Dec. 28, 2006 |

### 2) Tissues Used

Formalin-fixed, paraffin-embedded specimens of prostate cancer biopsies taken by fine needle testing

### 3) Method

At the two trial sites, among formalin-fixed, paraffin-embedded pathological tissue specimens, diagnosed as prostate cancer, which were taken and stored by fine needle testing during the period of 1982 to 2000, cases designated as Stage II to Stage IV by histopathological diagnoses were randomly extracted and subjected to immunohistochemical staining according to Method for Operation described above using the present kit. The results of staining were observed and rated as scores according to Method for Determination described above and were analyzed for relevance between the results of staining (LAT1 scores) and five-year survival rate using the Kaplan-Meier method.

### (3) Results

### 1) Number of Cases and Breakdown of Results of Staining

For the cases diagnosed with prostate cancer, the determination kit of Production Example 2 was used to study the expression of LAT1 protein in tumor cells according to immunohistochemical staining to observe staining with Scores 0 to 3 depending on the cases. The breakdown of the LAT1 scores at each site is shown in the table below. Classifying the results of all the cases by the score, both the institutions A and B tended to have cases with Score 0 in the largest numbers and cases with Score 3 in smaller numbers. Their staining behaviors were almost similar so that it was determined that there was no site-to-site difference in staining by the determination kit of Production Example 2.

**Table 11 Scoring of LAT1 protein expression in prostate cancer tissues**

| | Institution A | Institution B | Total |
|---|---|---|---|
| Score 0 | 87 (53.0) | 7 (71.0) | 92 (48.2) |
| Score 1 | 34 (20.7) | 0 (0) | 34 (19.9) |
| Score 2 | 25 (15.2) | 2 (28.6) | 27 (15.8) |
| Score 3 | 18 (11.0) | 0 (0) | 18 (10.5) |
| Total | 164 | 7 | 171 |

| | | | |
|---|---|---|---|
| Unit: number of cases. Numbers in parentheses denote percentages (%) on the basis of the total number of specimens at each site. | | | |

### 2) Survival Analysis in LAT1 Score Using Kaplan-Meire Method

For the cases of 1), the results at both the sites were integrated to analyze the relationship between the degree of expression of LAT1 protein in tumor cells (LAT1 scores) and the survival times of the case patients using the Kaplan-Meire method (non-parametric method) to observe a significant difference between the high LAT1 expression group (LAT1 Score 3) and the low LAT1 expression group (LAT1 Scores 0 to 2). Specifically, P<0.001 was obtained as a result between Score 3 and Score 0. Based on the result, for LAT1 Score 3, approximately half the number had an outcome of death within five years, showing a significant difference in relation to the case group with Score 0 (Fig. 7).

### 3) Conversion Analysis of LAT1 Score Using Sinicrope Method

For all the cases of 1), in addition to intensity of staining, coverage of staining was observed in three stages and classified into focal (10% or lower), partial (10 to 30%) and diffuse (30% or higher). For each observation result, the LAT1 score was multiplied by a factor corresponding to the coverage of staining (1 for focal, 2 for partial and 3 for diffuse) to convert the LAT1 score according to the Sinicrope method (Fig. 8). The converted LAT1 scores were analyzed in a similar manner to 2) using the Kaplan-Meier method, to observe a clearer difference between LAT1 score 1 and the survival times. Based on the result, at Scores 4 or higher, significant differences were observed in relation to Score 0. In particular, for cases with Scores 6 or higher, 99% had an outcome of death within five years. Thus, it was suggested that more accurate diagnoses be made possible by carrying out survival analyses using LAT1 scores (0 to 9) as converted by multiplying the intensity of the LAT1 scores by the expression coverage factors (focal=1, partial=2 and diffuse=3).

### (3) Comparison with PSA

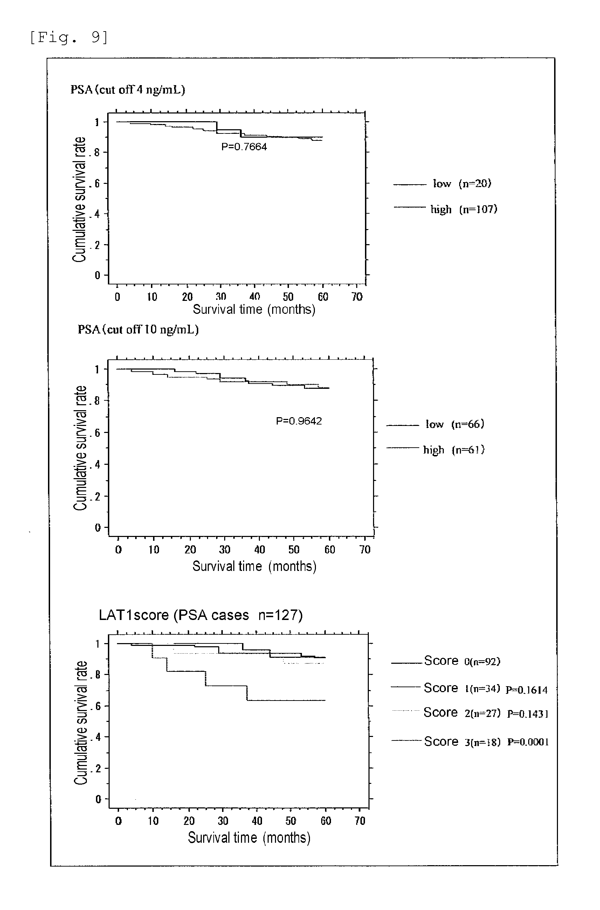

For similar cases, comparison was made with PSA, known as a prostate cancer-specific marker. Among 171 cases used for this occasion, for 127 cases whose PSA values were recorded immediately before surgical operations, survival analyses were carried out using the Kaplan-Meier method, with 4 ng/mL and 10 ng/mL PSA as cutoff values, to compare the results with those by the LAT1 scores. At either cutoff value, no significant difference in PSA value was observed between the low expression and high expression groups. By the LAT1 score analyses carried out using similar cases, a significant difference was observed between the Score 0 group and the Score 3 group (Fig. 9).

### (4) Comparison with Stage Classification

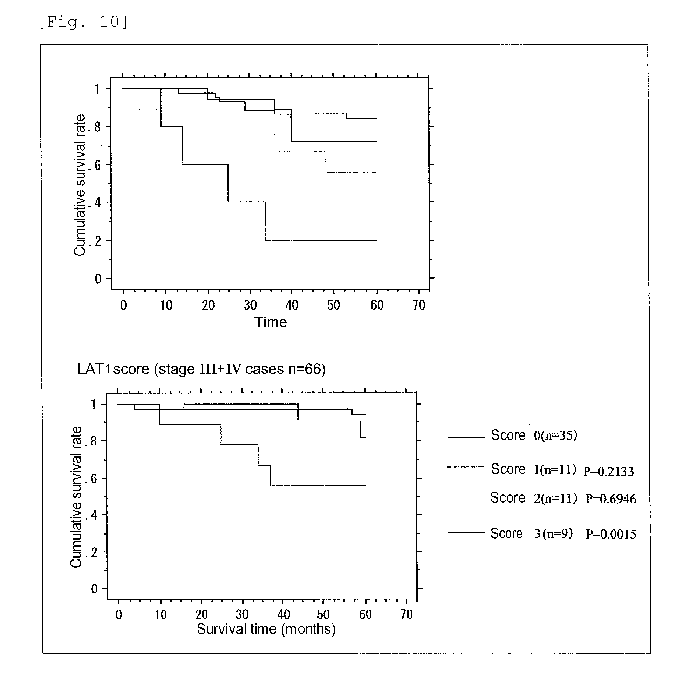

Among the case groups, based on the information during surgical operations, the Stage classification was carried out on the basis of the pathological TMN classification and the information concerning the presence or absence of metastases during the surgical operations to carry out the Kaplan-Meire survival analyses. By the Stage classification, no significant difference was observed in five-year survival rate, while by the LAT1 classification, a significant difference was observed between the Score 0 group and the Score 3 group at each stage (Fig. 10).

### (5) Comparison with Gleason Score

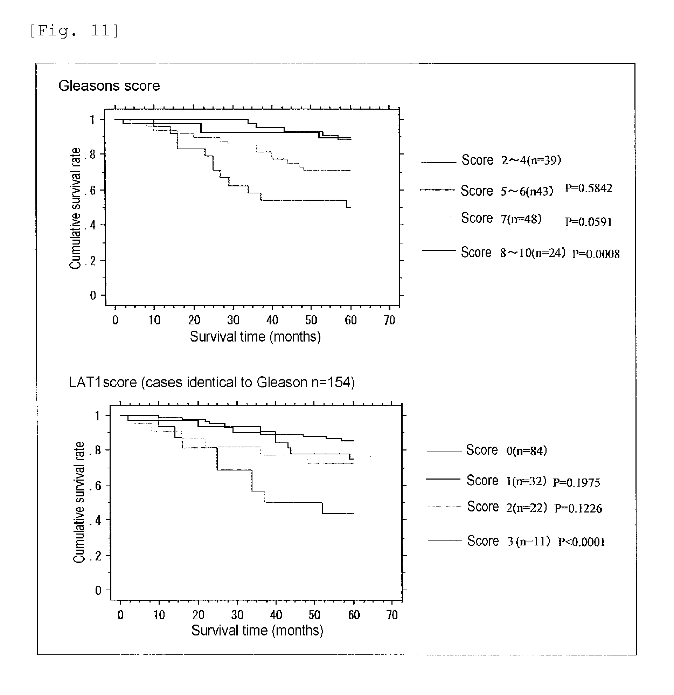

From a report on pathological diagnosis results of biopsies used in this testing, Gleason scores at the time of diagnoses were investigated and survival analyses were carried out according to the Kaplan-Meire method on 154 cases for which information was obtained, to compare with the results by LAT1 scores to obtain approximately equivalent results in both (Fig. 11). Thus, usefulness was demonstrated, which is equivalent to the results from the Gleason score classification (P=0.0008) that is valued the most in prostate cancer diagnosis.

### (6) Conclusion

Based on the clinical trial results, it was suggested that the expression of LAT1 be significantly inversely correlated to the survival times of prostate cancer patients and that its usefulness is equivalent to Gleason score. Also, excellent results were obtained in diagnosis of malignancy of cancers in comparison with PSA which is currently used as a marker for prostate cancer and the Stage classification which is supposed to indicate progression of cancers.

### INDUSTRIAL APPLICABILITY

It has been illustrated that the present procedure has good staining sensitivity to prostate tumor cells, with no non-specific reactions observed for characteristics of antibodies and no site-to-site difference.

Also, it is suggested that, by using the present procedure to qualitatively visualize the amount of LAT1 expressed in prostate tumor membranes, diagnosis of malignancy of prostate cancer cells may be made. It is further believed that its usefulness is equivalent to Gleason score and useful in comparison with PSA and the Stage classification. Furthermore, the present method qualitatively diagnoses LAT1 on membranes by means of immunohistochemical staining, so that the observation and determination of results may be easy in comparison with the Gleason's classification and other pathological diagnoses.

Further, it is expected that more accurate malignancy diagnosis of prostate cancer may be realized by combination of the present procedure with Gleason score. In particular, in diagnoses according to the Gleason's classification and the TMN classification, when a Gleason score reached approximately 7 in total, or when T3N0 (so-called Stage II) was determined, it is occasionally difficult to prognosticate; however, use in combination with the procedure according to the present invention contributes to clarify such gray areas.

Therefore, it is safe to say that the present procedure is a new diagnostic tool for prostate cancer in which malignancy of prostate cancer is diagnosed to prognosticate and determine subsequent therapeutic strategy.

### BRIEF DESCRIPTION OF THE DRAWINGS

Fig. 1 is a view showing prostate cancer cells being stained in human living cells.
Fig. 2 shows examples of determination on results of staining by the diagnostic method according to the present invention.
Fig. 3 is a view showing results of specificity testing.
Fig. 4 shows results of sensitivity testing.
Fig. 5 shows results of simultaneous reproducibility testing.
Fig. 6 shows results of confirmation of staining capability by an anti-LAT1 antibody using various specimens.
Fig. 7 shows results of survival analysis in LAT1 scores using the Kaplan-Meire method.
Fig. 8 shows relationship between LAT1 scores and survival times according to the Sinicrope method.
Fig. 9 shows results of comparison between PSA and LAT1 scores.
Fig. 10 shows results of comparison in relation to the Stage classification.
Fig. 11 shows results of comparison in relation to Gleason score.

## Claims

1. A kit, comprising an anti-human LAT1 monoclonal antibody, used to determine prostate cancer malignancy via immunohistochemical staining.

2. The kit used to determine prostate cancer malignancy according to Claim 1, wherein the monoclonal antibody recognizes human LAT1 amino acid residues specifically at positions 1 to 52 from the N-terminus.

3. A method for determining prostate cancer malignancy by means of immunohistochemical staining, which comprises a step of applying an anti-human LAT1 monoclonal antibody to a specimen tissue.

4. The method for determining prostate cancer malignancy according to Claim 3, wherein the monoclonal antibody recognizes human LAT1 amino acid residues specifically at positions 1 to 52 from the N-terminus.

5. A method to clinically differentiate prostate cancer severity via application of LAT1 molecular target therapeutic agent(s), which comprises a step of determining malignancy of prostate cancer according to the method as claimed in Claim 3 or 4 and a step of determining whether a therapeutic agent for prostate cancer is to be administered or not, based on the diagnosis result.
